# EUROPÄISCHE PATENTSCHRIFT

(11) **EP 2 659 876 B1**
(45) Veröffentlichungstag und Bekanntmachung des Hinweises auf die Patenterteilung: **24.10.2018**
(21) Anmeldenummer: 13158216.5
(22) Anmeldetag: 07.03.2013
(51) Int. Cl.: A61K 8/34, A61Q 5/06, A61K 8/04

(54) **Neue Haarfestigungsmittel mit Sorbitol**
New hair fixative with sorbitol
Nouvel agent de fixation capillaire contenant du sorbitol

(30) Priorität: 14.03.2012 DE 102012203942
(43) Veröffentlichungstag der Anmeldung: 06.11.2013
(73) Patentinhaber: Beiersdorf AG, 20253 Hamburg (DE)
(72) Erfinder: Sors, Nathalie, 21643 Beckdorf (DE); Klauck, Robert, 21465 Reinbek (DE); Krieger, Wiebke, 20259 Hamburg (DE); Fey, Sven, 22397 Hamburg (DE)

(56) Entgegenhaltungen:
- EP-A1- 1 642 561
- EP-A1- 2 420 220
- WO-A1-2006/097514
- WO-A1-2011/021681
- WO-A1-2011/025024
- DE-A1- 10 354 015
- JP-A- 2012 020 946

## Beschreibung

Natürliches Kopfhaar ist entscheidend für das Erscheinungsbild und Selbstwertgefühl des Individuums. Haar hängt zumeist glanzlos und schlaff ohne Volumen herab. Zur temporären Verformung und Stabilisierung der Frisur werden Produkte eingesetzt, die als Stylingmittel bekannt sind. Hierzu zählen Haarsprays, Schaumfestiger, Gele, Wachse u.s.w. Die Rezepturen sind vielfältig. Die Bestandteile müssen in ethanolischem, wässrigethanolischem oder wässrigem Medium verträglich sein.

Bei Haarstylingprodukten steht die fixierende Wirkung auf die gestaltete Frisur im Vordergrund. Ein wichtiger weiterer Aspekt ist die Glanzgebung, da Glanz mit gesunden Haaren verbunden wird. Üblicherweise werden Silikone als Glanzgeber eingesetzt. Diese sind jedoch Plastiziser und führen somit als Weichmacher zu einem weicheren Polymerfilm. Eine geringere Festigungs- und Halteleistung sind die Folge.

Wichtige Inhaltsstoffe dieser Haarfestiger stellen Filmbildner und Lösungsmittel dar. Üblicherweise bestehen diese Mittel zur Festigung der Haare aus Lösungen von filmbildenden natürlichen oder synthetischen Polymeren oder Polymerkombinationen. Es handelt sich dabei um nichtionische, anionische, kationische oder amphotere Polymere. Diese hinterlassen auf dem Haar nach der Applikation und nach Verdunstung des Lösungsmittels einen transparenten, farblosen Film, der die Fasern fixiert. Es werden eine Reihe von Anforderungen an diese Filme gestellt: Sie sollen klar, glänzend, feuchtigkeitsunempfindlich, festigend, langanhaltend, flexibel und nicht klebend sein, den natürlichen Griff des Haares nicht beeinträchtigen und sich auch wieder leicht durch handelsübliche Haarwaschmittel auswaschen lassen. Haarfestiger besitzen in der Gruppe der Stylingprodukte eine Sonderstellung. Nicht nur die Filmeigenschaften (Halt, Flexibilität...) stehen im Vordergrund, sondern auch die Pflegeleistung am Haar (Kämmbarkeit, gepflegter Griff, Glanz). Bei Schaumfestigern hat außerdem die Schaumcharakteristik (feinblasig, cremig, stabil) einen beträchtlichen Einfluss auf die Produktperformance. Üblicherweise enthalten Stylingmittel weitere kosmetische Hilfsstoffe mit unterschiedlichen Funktionen wie Pflegestoffe, Konservierungsmittel, Parfüme, Emulgatoren, Treibmittel, weichmachende, anfeuchtende und/oder feuchthaltende Substanzen, Fette, Öle, Wachse, Fettalkohole, Fettsäureester oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Die Schrift WO 2007099271 A2 offenbart den Einsatz von Zuckeralkohol mit Gelbildner und Acrylat-Polymer.

Die Schrift EP 1813265 A1 offenbart den Einsatz von Acrylat-Verdcker, fixierendem Polymer und Polyol.

Die Schrift JP 201102180 A1 offenbart den Einsatz von 3-30% Polyalkylenglycol oder Zuckeralkohol mit spezifischen Angaben zur Klebrigkeit und Viskosität des Stylingproduktes.

Die Schrift WO 2010052876 A1 offenbart den Einsatz von einem filmbildenden Polymer mit einem Zucker oder dessen Alkohol, Wachs sowie einer Ölkomponente.

Die Schrift WO 2011021681 A1 offenbart den Einsatz eines Resin mit Sorbitol oder PEG.

Die Schrift EP 1595530 B1 offenbart den Einsatz eines grenzflächenaktiven Rohstoffes mit Monocarbonsäure, Polyol und über 1% eines weiteren Salzes.

Die Schrift WO 2011024300 A1 offenbart den Einsatz eines Phosphortensides, eines nichtionischen Tensides, Polyol, Öl und Neutralisationsmittel.

Die Schrift EP 1420758 B1 offenbart den Einsatz von über 10% Polysaccharid mit über 15% Sorbitol, PVOH oder PVP.

Die Schrift WO 2011025024 A1 offenbart den Einsatz über 1,5% eines festen Tensides, einen festen Zuckeralkohols oder Zuckers oder PEG-Polymer oder Salz, einen flüssigen Zuckeralkohols sowie einen Filmbildner.

Die Schrift DE 10354015 A1 offenbart ein Wasserglas enthaltendes, verdicktes Haarbehandlungsmittel.

All dies konnte die Mängel des Standes der Technik nicht beseitigen.

Es war eine Aufgabe der vorliegenden Erfindung, den Nachteilen des Stands der Technik abzuhelfen und Haarfestiger zu entwickeln, deren beanspruchte Produktperformance bzgl. Festigungs- und Halteleistung sowie die glanzsteigernden Eigenschaften zu optimieren und beides in Einklang zu bringen. Dabei sollte die Optimierung der Glanzleistung nicht zu einer Verschlechterung der entscheidenden Stylingleistung wie Festigung und Halt führen, sondern vielmehr die Stylingleistung verbessern. Bislang ging schließlich eine Verbesserung der Glanzeffekte oft mit einer Reduktion der Stylingleistung einher.

Überraschend und für den Fachmann nicht vorhersehbar hat sich nun herausgestellt, dass eine haarfestigende polymerhaltige Zubereitung gemäß den vorliegenden Ansprüchen, die aus einem Behälter in Form eines Schaums entnommen werden kann, enthaltend Sorbitol den Mängeln des Standes der Technik abhilft. Solche erfindungsgemäßen Zubereitungen ermöglichen sowohl eine verbesserte Festigungs- und Halteperformance als auch gesteigerte Glanzeigenschaft. Generell liefern Polyole jedoch nicht derartige Effekte. Dies wird im weiteren Verlauf am Beispiel von Schaumfestigern enthaltend PEG-8 gegenüber Schaumfestiger mit Sorbitol bzw. Placebo-Schaumfestigern gezeigt. Es ist bevorzugt, wenn die Zubereitung, aus einem Behälter unter dem Druck eines Treibmittels entnommen werden kann. Besonders bevorzugt ist es, wenn die Zubereitung ein Schaumfestiger, ganz besonders bevorzugt ein Aerosol-Schaumfestiger ist. Bevorzugt ist es, wenn gegebenenfalls der Gehalt an Treibgas zwischen 5 und 50 Gew.%, besonders bevorzugt zwischen 5 und 15 Gew.%, ganz besonders bevorzugt zwischen 8 und 10 Gew.% liegt. Bevorzugt ist als Treibgas ein Kohlenwasserstofftreibgas, besonders bevorzugt Propan-Butan-Mischungen. Bevorzugt ist es, wenn der Gehalt an Sorbitol höchstens 10 Gew.% beträgt, bezogen auf den Sorbitol-Aktivgehalt. Besonders bevorzugt ist es, wenn der Gehalt an Sorbitol 2 bis 8 Gew.%, ganz besonders bevorzugt 2,5 bis 6 Gew.% beträgt. Die erfindungsgemäße Zubereitung enthält Polymere mit Vinylpyrrolidon und Vinylacetat als Monomerbausteine. Dabei beträgt das bevorzugte molare Verhältnis von Vinylpyrrolidon zu Vinylacetat 3 zu 2 bis 4 zu 1, besonders bevorzugt 1 zu 1 bis 4 zu 1. Darüber hinaus ist der Einsatz von kationischen Polymeren von Vorteil. Besonders bevorzugt sind PQ-4, PQ-68 und /oder PQ-16 Vorteilhaft ist es, wenn der Gehalt an kationischen Polymeren bezogen auf ihren Aktivgehalt 0,01 bis 5 Gew.%, bevorzugt 0,03 bis 3,5 Gew.%, besonders bevorzugt 0,05 bis 1 Gew.% beträgt. Vorteilhaft ist es, wenn der Gehalt an kationischen Tensiden 0 bis 2 Gew.%, bevorzugt 0 bis 1 Gew.% besonders bevorzugt 0 bis 1 Gew.% beträgt.. Wenn kationische Tenside enthalten sind, so ist es vorteilhaft, wenn es sich um Cetrimoniumchlorid und/oder Hydoxyethyl Cetyldimonium Phosphate handelt. Hingegen zeigten andere Inhaltsstoffe keine Vorteile für die Frisurengestaltung und folglich ist es von Vorteil, ihren Einsatz zu beschränken. Hierzu zählen Silikonverbindungen, bevorzugt Cyclomethicone, Dimethicone und/oder PEG-1 Dimethicone, sowie Polysachcharide, deren Gehalt jeweils 0 bis max. 0,001 Gew.% betragen sollte. Auch der Gehalt an Metallkationen beträgt höchstens 0,5 Gew.%, der Gehalt an Tensiden und Emulgatoren, bevorzugt Ceteareth-20, höchstens 3 Gew.%. Davon beträgt der Gehalt an Tensiden mit einem HLB-Wert von 9 bis 16 höchstens 2 Gew.%. Der erfindungsgemäße Gehalt an Metallkationen beträgt höchstens 0,5 Gew.%, bevorzugt höchstens 0,2 Gew.% und ist bevorzugt bezogen auf mehrwertige Metallkationen, besonders bevorzugt auf mehrwertige Übergangsmetallkationen. Der pH-Wert liegt im Intervall 3,5 bis 6,5, bevorzugt im Intervall 4 bis 6 besonders bevorzugt im Intervall 4,5 bis 5,5. Zusätzlich können erfindungsgemäße Zubereitungen Vitamine, bevorzugt Niacinamide oder Panthenol, den pH-Wert regulierende Rohstoffe, bevorzugt Citronensäure, Aminomethylpropanol, Natronlauge und/oder Triethanolamin, Salze, bevorzugt Natriumchlorid, Kaliumchlorid, Cetrimoniumchlorid oder Hydoxyethyl Cetyldimonium Phosphate, Komplexbildner, bevorzugt EDTA, kationische Polymere, bevorzugt Polyquaternium-4, Polyquaternium-16 und/oder Polyquaternium-68, Konservierungsmittel, bevorzugt Natriumbenzoat, Phenoxyethanol, Parabene und/oder DMDM Hydantoin, Lösungsvermittler, bevorzugt PEG-40 Hydrogenated Castor Oil enthalten. Die Erfindung umfasst auch die Verwendung einer erfindungsgemäßen Zubereitung zur Frisurgestaltung. Die Erfindung umfasst ebenso die Verwendung von Sorbitol in einer erfindungsgemäßen Zubereitung zur Verbesserung von Glanzeigenschaften des mit der Zubereitung behandelten Haares und /oder zur Verbesserung des Haltes der Frisur von mit der Zubereitung behandelten Haaren. Weiterhin umfasst die Erfindung die Verwendung eines Aerosol-Schaumfestigers enthaltend a) Treibgas mit einem Gehalt von 5 und 50 Gew.%, besonders bevorzugt von 5 und 15 Gew.%, ganz besonders bevorzugt von 8 und 10 Gew.%, b) Sorbitol mit einem Gehalt von höchstens 10 Gew.%, besonders bevorzugt 2 bis 8 Gew.%, ganz besonders bevorzugt 2,5 bis 6 Gew.%, c) Copolymer mit den Monomerbausteinen Vinylpyrrolidon und Vinylacetat mit einem molaren Verhältnis von Vinylpyrrolidon zu Vinylacetat 1 zu 1 bis 4 zu 1, d) 0,01-5 Gew.% aktiv kationische Polymere, e) 0 bis 2 Gew.%, bevorzugt 0 bis 1 Gew.% kationische Tenside, f) höchstens 3 Gew.% an Tensiden und Emulgatoren, g) höchstens 2 Gew.% Tensiden mit einem HLB-Wert von 9 bis 16 bei einem pH-Wert der Lösung von 3,5-6,5, bevorzugt 4,0-6,0, besonders bevorzugt 4,5 bis 5,5 zur Frisurengestaltung und -fixierung, wobei die Glanzeigenschaften des mit der Zubereitung behandelten Haares verbessert werden und /oder der Frisurenhalt von mit der Zubereitung behandelten Haaren gesteigert wird.

Als haltgebende Polymere in Haarfestigern können statistische Copolymere Verwendung finden. Copolymerisiert werden in der Regel verschiedene nichtionische, anionische oder kationische / kationogene Monomeren mit Vinyl- oder Acryl-Funktion, wie z.B. Vinyllactame (Vinylpyrrolidon, Vinylcaprolactam), Vinylacetat, Alkyl(meth)acrylate (Methyl(meth)acrylat, Ethyl(meth)acrylat, Propyl(meth)acrylat, n-Butyl(meth)acrylat, tert-Butyl(meth)acrylat, Cyclohexyl(meth)acrylat, usw.), Styrol, (Meth)Acrylamid, Alkyl(meth)acrylamide, funktionalisierte Alkyl(meth)acrylate (z.B. Hydroxyethyl(meth)acrylat, Dimethylaminoethyl(meth)acrylat), Dimethylaminopropyl(meth)acrylat), funktionalisierte Alkyl(meth)acrylamide (Dimethylaminopropyl(meth)acrylamid), (Meth)Acrylsäure, Maleinsäure, Crotonsäure, Acrylamidomethylpropansulfonsäure, aminogruppenhaltige Monomere, deren Aminogruppe alkyliert (quarternisiert) vorliegt, wie (Meth)Acryloylethyltrialkyl Ammoniumchlorid, (Meth)Acryloylpropyltrialkyl Ammoniumchlorid, (Meth)Acryloylaminopropyltrialkyl Ammoniumchlorid, (Meth)Acryloylethyltrialkyl Ammoniummethosulfat, (Meth)Acryloylpropyltrialkyl Ammoniummethosulfat, (Meth)Acryloylaminopropyltrialkyl Ammoniummethosulfat. Beispiele für in Haarfestigern verwendete Polymere dieser Art sind z.B. Polymere mit der INCI Acrylates Copolymer (Luvimer®, Luviflex® Soft von BASF, Balance® von AkzoNobel, Avalure AC® von Lubrizol), Acrylates/Hydroxyesters Acrylates Copolymer (Acudyne® von Dow Chemical), Acrylates/Octylacrylamide Copolymer (Amphomer® oder Resyn XP von AkzoNobel), AMP-Acrylates/Allyl Methacrylates Copolymer (Fixate® von Lubrizol), Butyl Ester of PVM/MA Copolymer (Gantrez® von Ashland Speciality Ingredients), Ethyl Ester of PVM/MA Copoylmer (Gantrez®, Omnirez® oder Advantage® von Ashland Speciality Ingredients), Acrylamide/Sodium Acryloyldimethyltaurate/Acrylic Acid Polymer (Acudyne SCP® von Dow Chemical), Isobutylene/Ethylmaleimide/Hydroxyethylmaleimide Copolymer (Aquaflex FX-64® von Ashland Speciality Ingredients), Octylacrylamide/Acrylates/Butylaminoethyl Methacrylate Copolymer (Amphomer® oder Balance® von AkzoNobel), Acrylates/t-Butylacrylamid Copolymer (Ultrahold® von BASF), PEG/PPG-25/25 Dimethicone/Acrylates/t-Butylacrylates Copolymer (Luviflex Silk® von BASF), Acrylic Acid/VP Crosspolymer (Ultrathix P-100® von Ashland Speciality Ingredients), Polyvinylcaprolactam (Luviskol PIus® von BASF), PVM/MA Copolymer (Luviform FA® von BASF), PVP (Luviskol® von BASF), PVP/VA Copolymer (Luviskol® von BASF), VP/Dimethylaminoethylmethacrylate Copolymer (Copolymer® von Ashland Speciality Ingredients), Sodium Polystyrene Sulfonate (Flexan® von AkzoNobel), VA/Crotonates/Vinyl Neodecanoate Copoylmer (Luviset CAN® von BASF, Resyn® von AkzoNobel), VA/Crotonates/Vinyl Propionate Copolymer (Luviset CAP® von BASF), VA/Butyl Maleate/Isobornyl Acrylate Copolymer (Advantage Plus® von Ashland Speciality Ingredients), VA/Crotonates Copolymer (Luviset CA 66® von BASF, Aristoflex® von Clariant), VA/Vinylbutylbenzoate/Crotonates Copolymer (Mexomere PW® von Chimex), Vinylcaprolactam/PVP/Dimethylaminoethyl Methacrylate Copolymer (Advantage® von Ashland Speciality Ingredients), VA/Butyl Maleate/Isobornyl Acrylate Copolymer (Advantage Plus® von Ashland Speciality Ingredients), Acrylates/C1-2 Succinates/HydroxyAcrylates Copolymer (Allianz LT-120® von Ashland Speciality Ingredients), VP/Vinyl Caprolactam/DMAPA Acrylates Copolymer (Aquaflex SF-40® von Ashland Speciality Ingredients), Vinyl Caprolactam/VP/Dimethylaminoethyl Methacrylate Copolymer (Gaffix® von Ashland Speciality Ingredients), VP/DMAPA Acrylates Copolymer (Styleze CC-10 von Ashland Speciality Ingredients), VP/Acrylates/Lauryl Methacrylate Copolymer (Styleze 2000® von Ashland Speciality Ingredients), VP/Methacrylamide/N-Vinyl Imidazole Copolymer (Luviset Clear® von BASF), alle Polyacrylat-Typen (Fixate Plus® und Fixate Superhold von Lubrizol, Luviset Shape® von BASF) und viele mehr. Weitere Polymerklassen, die in Haarfestigern Einsatz finden sind z.B. Polyurethane, z.B. Polyurethane-1 (Luviset PUR® von BASF), Polyurethane-2 (Avalure UR® von Lubrizol), Polyurethane-6 (Luviset SI PUR® von BASF), Polyurethane-14 (and) AMP-Acrylates Copolymer (DynamX von AkzoNobel), Polyester, z.B. Polyester-5 (AQ-Polymere von Eastman), Polyimide, z.B. Polyimid-1 (Aquaflex XL-30® von Ashland Speciality Ingredients), und viele mehr.

Kationische Tenside werden zumeist ausgewählt aus der Gruppe der quaternären Ammoniumverbindungen, insbesondere Benzyltrialkylammoniumsalze oder Alkyltrimethylammoniumsalze, beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Hydroxyethyl Cetyldimonium Phosphate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide.

Kationische Polymere sind unter den Bezeichnungen Polyquaternium beschriebene Polymere wie quaternisierte Copolymere von Vinylimidazol, Vinylpyrrolidon und/oder Vinylcaprolactam (Polyquaternium-16, -44 oder -46), quaternisiertes Vinylpyrrolidon/-Dimethylaminoethylmethacrylat Copolymer (Polyquaternium-11), Homo- und Copolymere von Dimethyldiallylammoniumchlorid (Polyquaternium-6, -7 oder -22), quaternisierte Cellulosepolymere (Polyquaternium-4, -10), Copolymere aus kationischer Cellulose und Stärke (Polyquaternium-4 / Hydroxypropyl Starch Copolymer), quaternisiertes Vinylpyrrolidon / Dimethylaminopropylmethacrylat / Lauryl Dimethylaminopropylmethacrylat Copolymer (Polyquaternium-55), Vinylpyrrolidon/ Vinylimidazol/Methacrylamid/quaternisiertes Vinylimidazol (Polyquaternium-68), quaternisiertes Vinylcaprolactam/Vinylpyrrolidone/Dimethylaminopropyl methacrylamide /Methacryloylaminopropyllauryldimoniumchloride (Polyquaternium-69) oder quaternisierte Guarderivate.

Zur Verbesserung der Schaumeigenschaften ist der Zusatz von schaumstabilsierenden Additiven, wie z.B. nichtionischen Tensiden üblich. Als nicht-ionische Tenside werden üblicherweise eingesetzt Alkohole, Alkanolamide, wie Cocamide MEA/ DEA/ MIPA, Aminoxide, wie Cocoamidopropylaminoxid, Ester, die durch Veresterung von Carbonsäuren mit Ethylenoxid, Glycerin, Sorbitan oder anderen Alkoholen entstehen, Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid, Sucroseester, -ether, Polyglycerinester, Diglycerinester, Monoglycerinester, Methylglucosester, sowie Ester von Hydroxysäuren.

Als Treibmittel dienen üblicherweise Kohlenwasserstoffe wie Butan, Isobutan und Propan, Dimethylether oder Fluorkohlenwasserstoffe wie z.B. 1,2-Diflourethan. Andere Treibmittel sind Stickstoff, Kohlendioxid, Distickstoffoxid oder komprimierte Luft.

### Beispiele

Nachfolgende Beispiele sollen die Erfindung verdeutlichen, ohne sie einzuschränken. Dabei geben die Konzentrationen die Gesamteinsatzmenge an, nicht den Aktivgehalt.

### Rezepturen

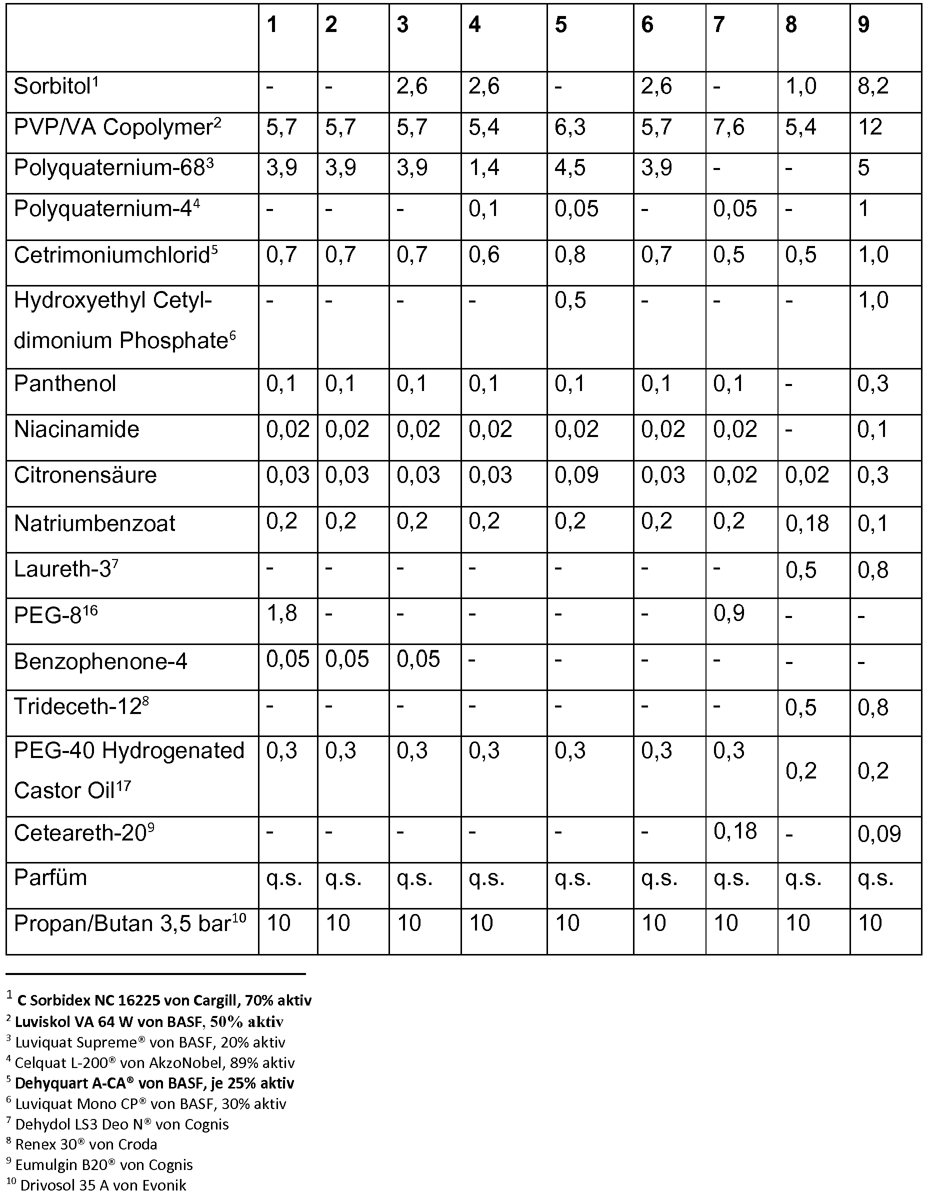

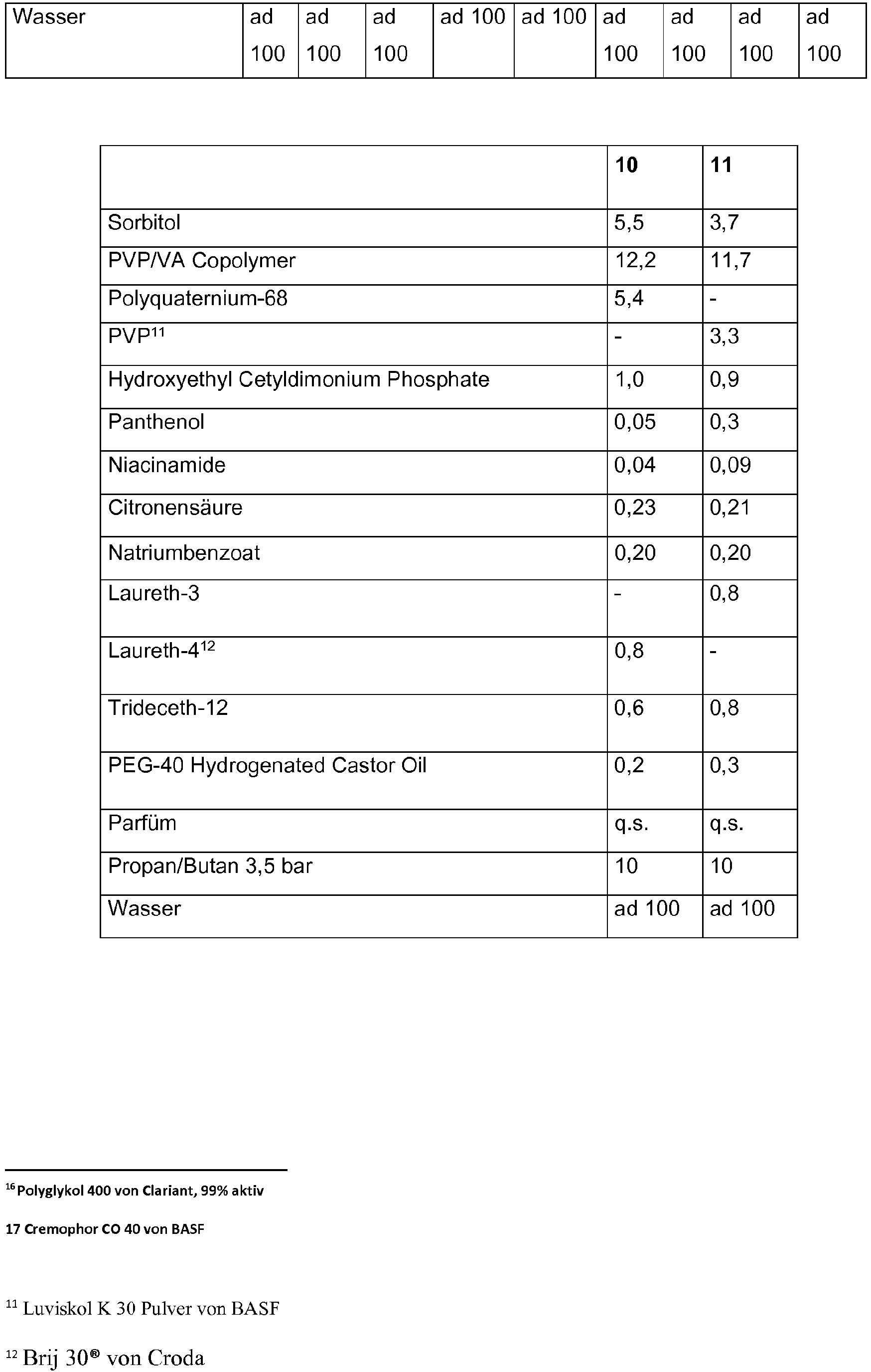

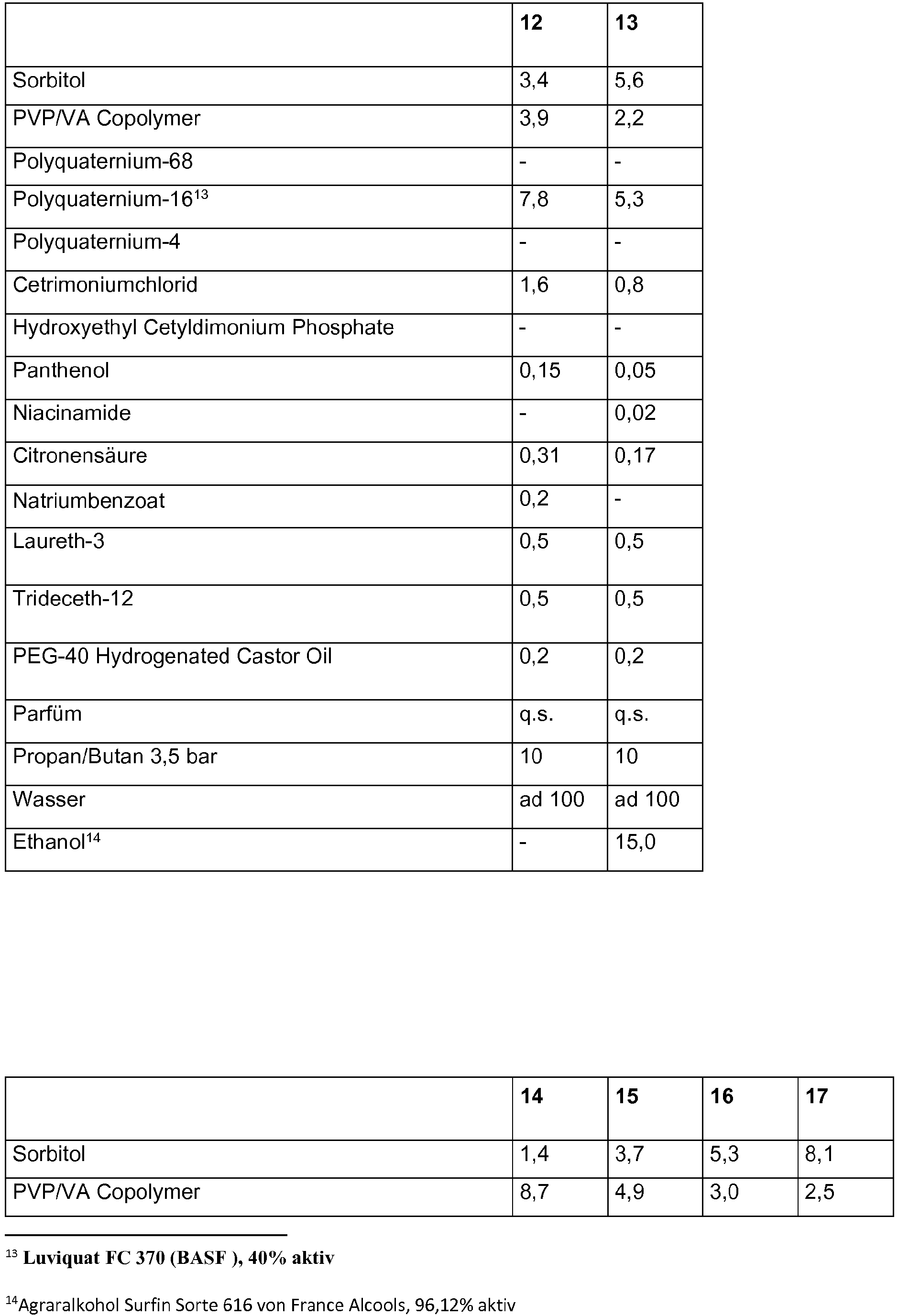

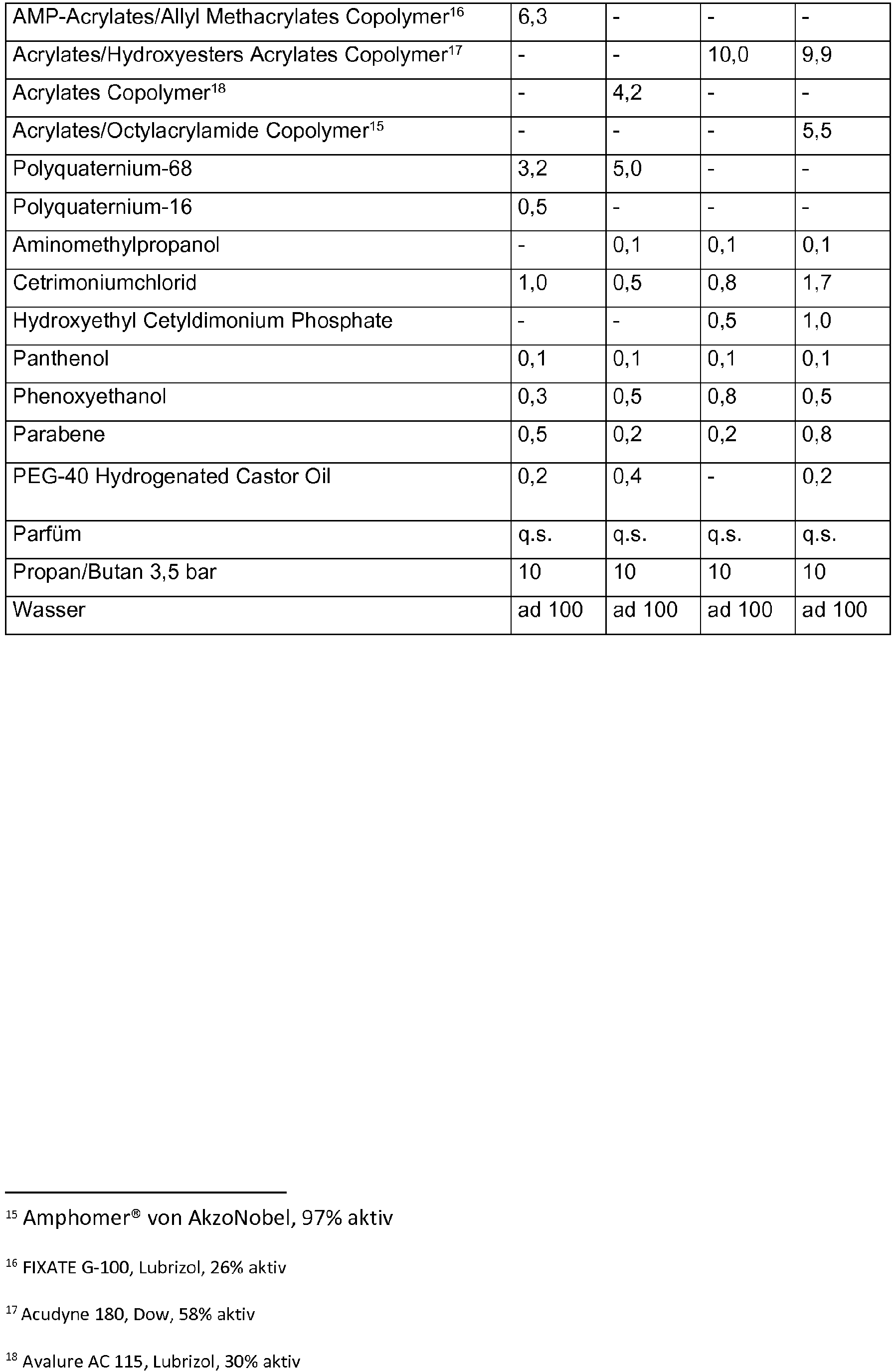

### A) Festigungs-/Halteergebnisse Sorbitol & PEG-8 in vitro:

Drei Schaumfestiger wurden auf nasse, ungebleichte Tressen aufgetragen und über Nacht bei Raumtemperatur getrocknet. Anschließend beurteilten Panelisten die Festigungsleistung der Produkte am Haar in einem Ranking von 1 (höchste Festigung) bis 3 (geringste Festigung).

**Tab 1: Ergebnisse Festigung/Halt**

| | **Bsp. 1** | **Bsp. 2** | **Bsp. 3** |
|---|---|---|---|
| Festigung | **PEG-8** | **Placebo** | **Sorbitol** |
| Mittelwert | 2.3 | 2 | 1.7 |

Das Produkt mit 2%-Aktivgehalt Sorbitol zeigte die beste Festigungsleistung gegenüber Placebo, 2%-Aktivgehalt PEG-8 hingegen die schlechteste. Das Polyol PEG-8 hat somit die Halteperformance verschlechtert, das Sorbitol-Polyol hingegen verbessert.

### B) Glanzergebnisse Sorbitol in vitro:

*In vitro* konnte eine deutliche Verbesserung des Glanzeffektes durch Sorbitol nachgewiesen werden. Als *in vitro*-Glanzmethode wurde die im Journal of the Society of Cosmetic Chemists, 2011, Vol. 62, No 5, 453 ff publizierte Methode an ungebleichten Haartressen eingesetzt.
Bsp. 2 (Placebo) wies einen Glanzwert von 0,81 auf,
Bsp. 3 (mit Sorbitol) wies einen Glanzwert vo 0,96 auf.

Der Schaumfestiger mit Sorbitol erhöht den Glanz signifikant gegenüber dem entsprechenden Placebo-Schaumfestiger ohne Sorbitol.

### C) Glanzergebnisse sowie Haltperformance von Sorbitol und PEG-8 in vivo:

Es wurden monadische Home-in-Use-Anwendungsstudien in Deutschland mit verblendeten Packmitteln und regelmäßigen Schaumfestigerverwenderinnen (über 160 Testerinnen pro Produktmuster) durchgeführt. Die Bewertung erfolgte anhand einer 7er-Skala nach der Anwendung.

**Tab. 2: Ergebnisse Glanz & Halt von Sorbitol und PEG-8**

| | Bsp. 5 Placebo | Bsp. 6 mit Sorbitol | Bsp. 7 mit PEG-8 |
|---|---|---|---|
| Gives your hair a silky shine | 5,4 | 5,6 | 4,8 |
| Gives your hair particularly good hold | 5,3 | 5,5 | 4,7 |

Der Schaumfestiger mit Sorbitol wurde bzgl. Glanz und Halt besser bewertet als der Schaumfestiger ohne Sorbitol. Der Schaumfestiger enthaltend PEG-8 reduzierte hingegen Glanz- und Halteeigenschaften.

## Patentansprüche

1. Aerosol-Schaumfestiger, **dadurch gekennzeichnet, dass** die Zubereitung Sorbitol mit einem Gehalt von höchstens 10 Gew.-%, Polymere mit Vinylpyrrolidon und Vinylacetat als Monomerbausteinen und Treibgas mit einem Gehalt zwischen 5 und 50 Gew.-% enthält,
wobei der Gehalt an Tensiden und Emulgatoren höchstens 3 Gew.-% beträgt.

2. Zubereitung nach Patentanspruch 1, **dadurch gekennzeichnet, dass** das molare Verhältnis von Vinylpyrrolidon zu Vinylacetat 3 zu 2 bis 4 zu 1 beträgt.

3. Zubereitung nach einem der vorangehenden Patentansprüche, **dadurch gekennzeichnet, dass** der Gehalt an Tensiden mit einem HLB-Wert von 9 bis 14 höchstens 2 Gew.% beträgt.

4. Zubereitung nach einem der vorangehenden Patentansprüche, **dadurch gekennzeichnet, dass** zusätzlich kationische Polymere enthalten sind.

5. Zubereitung nach einem der vorangehenden Patentansprüche, **dadurch gekennzeichnet, dass** die kationischen Polymere mit einem Gehalt von 0,01 bis 5 Gew.%, bevorzugt 0,03 bis 3,5 Gew.%, besonders bevorzugt 0,05 bis 1 Gew.%, bezogen auf ihren Aktivgehalt, vorliegen.

6. Zubereitung nach einem der vorangehenden Patentansprüche, **dadurch gekennzeichnet, dass** kationische Tenside mit einem Gehalt von 0 bis 2 Gew. %, bevorzugt 0 bis 1 Gew.-% besonders bevorzugt 0 bis 1 Gew. % enthalten sind.

7. Verwendung eines Aerosol-Schaumfestigers enthaltend
a. Treibgas mit einem Gehalt zwischen 5 und 50 Gew.%, besonders bevorzugt zwischen 5 und 15 Gew.%, ganz besonders bevorzugt zwischen 8 und 10 Gew.%,
b. Sorbitol mit einem Gehalt von höchstens 10 Gew.%, besonders bevorzugt 2 bis 8 Gew.%, ganz besonders bevorzugt 2,5 bis 6 Gew.%,
c. Copolymer mit den Monomerbausteinen Vinylpyrrolidon und Vinylacetat mit einem molaren Verhältnis von Vinylpyrrolidon zu Vinylacetat 1 zu 1 bis 4 zu 1,
d. 0,01-5 Gew.% aktiv kationische Polymere,
e. 0 bis 2 Gew.%, bevorzugt 0 bis 1 Gew.% kationische Tenside,
f. höchstens 3 Gew.% an Tensiden und Emulgatoren,
g. höchstens 2 Gew. % Tenside mit einem HLB-Wert von 9 bis 16 und
h. bei einem pH-Wert der Lösung 3,5-6,5, bevorzugt 4,0-6,0, besonders bevorzugt 4,5 bis 5,5
zur Frisurengestaltung und -fixierung, wobei die Glanzeigenschaften des mit der Zubereitung behandelten Haares verbessert werden und /oder der Frisurenhalt von mit der Zubereitung behandelten Haaren gesteigert wird.

## Claims

1. Aerosol mousse fixative, **characterized in that** the preparation comprises sorbitol at a content of at most 10 wt%, polymers with vinylpyrrolidone and vinyl acetate as monomer units, and propellant gas at a content of between 5 and 50 wt%,
wherein the content of surfactants and emulsifiers is at most 3 wt%.

2. Preparation according to Claim 1, **characterized in that** the molar ratio of vinylpyrrolidone to vinyl acetate is 3:2 to 4:1.

3. Preparation according to either of the preceding claims, **characterized in that** the content of surfactants having an HLB value of 9 to 14 is at most 2 wt%.

4. Preparation according to one of the preceding claims, **characterized in that** cationic polymers are additionally present.

5. Preparation according to one of the preceding claims, **characterized in that** the cationic polymers are present at a content of 0.01 to 5 wt%, preferably 0.03 to 3.5 wt%, particularly preferably 0.05 to 1 wt%, based on the active content thereof.

6. Preparation according to one of the preceding claims, **characterized in that** cationic surfactants are present at a content of 0 to 2 wt%, preferably 0 to 1 wt%, particularly preferably 0 to 1 wt%.

7. Use of an aerosol mousse fixative comprising
a. propellant gas at a content of between 5 and 50 wt%, particularly preferably between 5 and 15 wt%, very particularly preferably between 8 and 10 wt%,
b. sorbitol at a content of at most 10 wt%, particularly preferably 2 to 8 wt%, very particularly preferably 2.5 to 6 wt%,
c. copolymer having the monomer units vinylpyrrolidone and vinyl acetate having a molar ratio of vinylpyrrolidone to vinyl acetate of 1:1 to 4:1,
d. 0.01-5 wt% of active cationic polymers,
e. 0 to 2 wt%, preferably 0 to 1 wt% of cationic surfactants,
f. at most 3 wt% of surfactants and emulsifiers,
g. at most 2 wt% of surfactants having an HLB value of 9 to 16, and
h. at a pH value of the solution of 3.5-6.5, preferably 4.0-6.0, particularly preferably 4.5 to 5.5
for styling and fixing the hair, wherein the shine properties of the hair treated with the preparation are improved and/or the hold of the hair treated with the preparation is increased.

## Revendications

1. Mousse de fixation en aérosol, **caractérisée en ce que** la préparation contient du sorbitol en une teneur d'au plus 10 % en poids, des polymères contenant de la vinylpyrrolidone et de l'acétate de vinyle en tant que constituants monomères et un gaz propulseur en une teneur comprise entre 5 et 50 % en poids, la teneur en tensioactifs et en émulsifiants étant d'au plus 3 % en poids.

2. Préparation selon la revendication 1, **caractérisée en ce que** le rapport molaire entre la vinylpyrrolidone et l'acétate de vinyle est de 3 sur 2 à 4 sur 1.

3. Préparation selon l'une quelconque des revendications précédentes, **caractérisée en ce que** la teneur en tensioactifs ayant une valeur HLB de 9 à 14 est d'au plus 2 % en poids.

4. Préparation selon l'une quelconque des revendications précédentes, **caractérisée en ce que** des polymères cationiques sont en outre contenus.

5. Préparation selon l'une quelconque des revendications précédentes, **caractérisée en ce que** les polymères cationiques sont présents en une teneur de 0,01 à 5 % en poids, de préférence de 0,03 à 3,5 % en poids, de manière particulièrement préférée de 0,05 à 1 % en poids, par rapport à leur teneur en agent actif.

6. Préparation selon l'une quelconque des revendications précédentes, **caractérisée en ce que** des tensioactifs cationiques sont contenus en une teneur de 0 à 2 % en poids, de préférence de 0 à 1 % en poids, de manière particulièrement préférée de 0 à 1 % en poids.

7. Utilisation d'une mousse de fixation en aérosol, contenant :
a. un gaz propulseur en une teneur comprise entre 5 et 50 % en poids, de manière particulièrement préférée comprise entre 5 et 15 % en poids, de manière tout particulièrement préférée comprise entre 8 et 10 % en poids,
b. du sorbitol en une teneur d'au plus 10 % en poids, de manière particulièrement préférée de 2 à 8 % en poids, de manière tout particulièrement préférée de 2,5 à 6 % en poids,
c. un copolymère contenant les constituants monomères vinylpyrrolidone et acétate de vinyle en un rapport molaire entre la vinylpyrrolidone et l'acétate de vinyle de 1 sur 1 à 4 sur 1,
d. 0,01 à 5 % en poids de polymères activement cationiques,
e. 0 à 2 % en poids, de préférence 0 à 1 % en poids de tensioactifs cationiques,
f. au plus 3 % en poids de tensioactifs et d'émulsifiants,
g. au plus 2 % en poids de tensioactifs ayant une valeur HLB de 9 à 16,
h. à un pH de la solution de 3,5 à 6,5, de préférence de 4,0 à 6,0, de manière particulièrement préférée de 4,5 à 5,5,
pour le coiffage et la fixation de cheveux, les propriétés de brillance des cheveux traités avec la préparation étant améliorées et/ou la tenue des cheveux traités avec la préparation étant augmentée.
